Europäisches Patentamt

European Patent Office

Office européen des brevets

(11) Publication number: **0 183 215**
**B1**

(12) **EUROPEAN PATENT SPECIFICATION**

(45) Date of publication of patent specification: **18.07.90**

(21) Application number: **85114921.1**

(22) Date of filing: **25.11.85**

(51) Int. Cl.⁵: **G 01 N 33/571**

(54) **Method for determination of herpes simplex virus antibodies.**

(30) Priority: **26.11.84 JP 250343/84**

(43) Date of publication of application:
**04.06.86 Bulletin 86/23**

(45) Publication of the grant of the patent:
**18.07.90 Bulletin 90/29**

(84) Designated Contracting States:
**AT BE CH DE FR GB IT LI NL SE**

(56) References cited:
**EP-A-0 001 223**
**FR-A-2 187 909**
**US-A-3 562 384**
**US-A-4 130 395**
**US-A-4 403 037**
**US-A-4 529 712**

(73) Proprietor: **Juridical Foundation The Chemo-**
**Sero-Therapeutic Research Institute**
**668, Ohkubo Shimizu-machi**
**Kumamoto-shi Kumamoto-ken (JP)**

(72) Inventor: **Kino, Yoichiro**
**Kodan-apt. 3-1003 2-142, Musashigaoka**
**Kumamoto-shi Kumamoto-ken (JP)**
Inventor: **Mizuno, Kyosuke**
**1725-1, Kamitatsuta Tatsuta-machi**
**Kumamoto-shi Kumamoto-ken (JP)**

(74) Representative: **Vossius & Partner**
**Siebertstrasse 4 P.O. Box 86 07 67**
**D-8000 München 86 (DE)**

The file contains technical information
submitted after the application was filed and
not included in this specification

Note: Within nine months from the publication of the mention of the grant of the European patent, any person may
give notice to the European Patent Office of opposition to the European patent granted. Notice of opposition shall
be filed in a written reasoned statement. It shall not be deemed to have been filed until the opposition fee has been
paid. (Art. 99(1) European patent convention).

Courier Press, Leamington Spa, England.

**Description**

The present invention relates to a novel method for the determination of herpes simplex virus (hereinafter referred to as HSV) antibodies.

The recent increase in the number of cases of infectious diseases due to HSV, such as herpes genitalis, neonatal herpes infection and herpes encephalitis, has given rise to serious social problems. One of the reasons for the increase may be a decreased percentage of the population having antibodies against HSV. Almost every case of neonatal herpes occurs in babies born to mothers who do not have antibodies in their blood for preventing neonatal herpes. In the field of dermatology, where skin diseases due to HSV occur quite often, determination of antibodies against HSV is desirable in order to prevent HSV infections.

The ordinary methods for the determination of antibodies against HSV are the neutralization test, the complement fixation test (CFT), and the enzyme-linked immunosorbent assay (ELISA).

The neutralization test cannot, however, be conducted without cell-cultural instruments and countermeasures against biohazards since the virus being handled is infectious. In CFT, many reagents such as virus antigens, sheep blood cells, hemolysin and complement are required and the method is troublesome. ELISA is high in sensitivity, but requires very expensive instruments for the determination.

EP—A—0 001 223 discloses a generally applicable technique of an immunoassay which involves an agglutination method. In context with this general disclosure, a comprehensive list of potential applicabilities of this method is set forth in Table I on page 6 et seq. of EP—A—0 001 223. Furthermore, it is generally mentioned in the penultimate paragraph on page 5 of EP—A—0 001 223 that glycoproteins can be determined by using this allegedly generally applicable test.

However, it has to be appreciated that EP—A—0 001 223 does not disclose that HSV glycoprotein can be used for the determination of anti-HSV-antibodies in a sample and that such a determination is a reliable test of whether a given individual is or was HSV infected. Furthermore, EP—A—0 001 223 does not suggest that it is advantageous to use the HSV glycoproteins instead of the whole virus particles in an agglutination assay.

FR—A—2 187 909 also discloses a generally applicable agglutination test for biological substances. Moreover, it is mentioned in FR—A—2 187 909 that HSV-derived antigens can be used in the test. However, in this test, the envelope of HSV is removed before the thus obtained HSV-derived antigen is coupled to red-blood cells. Therefore, the HSV-derived antigen mentioned in FR—A—2 187 909 does not even contain HSV glycoproteins because these are removed together with the envelope.

Summing up, it is not suggested that HSV glycoprotein could be used in an agglutination assay as a reliable test of whether a given individual is or was HSV infected, let alone that the use of such HSV glycoproteins is advantageous in comparison to the use of whole HSV particles.

US—A—3 562 384 only discloses that microbial cells can be used as carriers for antigenic substances in agglutination assays for the detection of antibodies.

Thus, an inexpensive and speedy method not requiring special instruments is still required, particularly in cases where many samples have to be determined with respect to the presence of antibodies against HSV.

The inventors have studied methods for the determination of HSV antibodies with an aim to overcome the defects of the prior art and have established an effective assay by applying passive agglutination.

Four kinds of glycoproteins are present in HSV particles and the cell membrane of HSV-infected cells: $g^C$, $g^B$, $g^E$ and $g^D$. It is known that these glycoproteins serve as important target antigens in humoral immunity and cell-mediated immunity. The glycoproteins can easily be extracted from virus-infected cells and virus particles with particular surfactants.

Thus, using such extracted antigens, studies have been made on methods for the production of a subunit vaccine, and as a result, it has been proved that these glycoproteins are effective as infection-preventing antigens.

On the other hand, monoclonal antibodies against HSV glycoproteins have recently been produced. Studies on such monoclonal antibodies show that the antibodies have properties such as neutralization capacity, ADCC (antibody-dependent cellular cytotoxicity) and antibody-dependent complement-mediated cytolysis.

It has also been reported that the antibody titer against the glycoproteins is higher than that against non-glycoproteins, when determined with respect to blood of patients infected with HSV. This means that the antibodies against the glycoproteins of HSV play a more important role than antibodies against the other proteins.

It is considered that antibodies determined by the conventional methods such as the neutralization method, CFT and ELISA are substantially those against the glycoproteins which are specific to HSV.

Thus, according to the present invention, there is provided a new assay method for HSV utilizing passive agglutination in which there is employed a carrier sensitized with the glycoproteins of HSV, which have been purified.

In carrying out the present invention, Triton® X-100 (tradename for polyoxyethylene ether, manufactured by Rohm and Hass Co.), Nonidet® P-40 (tradename for octylphenoxypolyethyloxyethanol, manufactured by Shell Oil Company), Tween®-20 (tradename for polyoxyethylene sorbitan monolaurate, manufactured by Bio-Rad), deoxycholic acid, sodium dodecylsulfate and N-lauroylsarcosine sodium salt, preferably a nonionic surfactant for instance Triton® X-100 or Nonidet® P-40, can generally be used for extracting the HSV-specific glycoproteins which are generated by infection with HSV from a variety of cell membranes. The amount of such a surfactant to be added is usually 0.5 to 10 w/v%, preferably 1 to 2 w/v%.

Then the extracted HSV-specific glycoproteins are purified, for example, by being adsorbed and eluted from lentil lectin or concanavalin A or a carrier loaded with anti-HSV antibodies. The purified HSV glycoproteins are then used to irreversibly sensitize a suitable carrier, with a reagent such as tannic acid, to obtain a sensitized carrier for the passive agglutination reaction according to the present invention. As the carrier, there may, for example, be used mammalian or avain red blood cells fixed by glutaraldehyde or formalin, or artificial carriers such as latex (styrene polymer), gelatin, epoxy resins, cellulose resins, and activated charcoal powder.

The agglutination with the antigen-sensitized carrier thus prepared makes it possible to determine the titer against HSV antibodies as described in more detail below.

The method for the determination of HSV antibodies of the present invention, in which there is employed the passive agglutination with a carrier sensitized with the HSV-specific glycoproteins, is quite simple, and does not require sophisticated instruments. It is therefore much less expensive than the conventional methods. Further, the method of the present invention is advantageous in that the sensitized carrier can be stored for a long time and used at any time required. For example, the carrier can be stored in 1/15 M phosphate buffered saline (PBS: pH 7.2) containing lactose, serum albumin and sodium azide, or be stored as a lyophilized product.

Preparation 1

Fixation of sheep red blood cells:

100 ml of sheep blood was mixed with 100 ml of Alsever's solution. The resultant mixture was then subjected to filtration with gauze. The concentration of the blood cells was determined by measuring the hematocrit.

Following washing with saline five times, formalin-fixed sheep blood cells were prepared according to "Methods in Immunology and Immunochemistry", Vol. IV pp. 33—34 (1977), Abram B. Stavistsky, (edited by Williams, Chase Academic Press, New York) in the following manner:

After washing and centrifugation, to a precipitate of red blood cells was added eight times the volume of cold saline solution containing 3% formaldehyde and the mixture was stirred slowly at 4°C for 24 hours. Further, there was added a cold saline solution containing 40% formaldehyde, in twice the volume of the precipitate, and the resultant mixture was stirred for 24 hours. Then the red blood cells were filtered off using gauze and washed with saline eight times. The washed product was then suspended in PBS at a concentration of 2.5%. To this suspension was added sodium azide (1/100 w/v%) for storage at 4°C.

Preparation 2

Purification of HSV glycoproteins:

HSV-1 KOS strain was inoculated into human laryngeal cancer cells (HEp-2) at a Mol (multiplicity of infection) of 10. After incubation at 37°C for 1 hour, the strain was cultured for 18 hours in Dulbeco's modified Eagle's minimal (DEM) medium containing 5% fetal calf serum.

The HSV-infected cells were washed with PBS and solubilized in a suitable buffer solution (for example, 50 mM tris · HCl buffer solution at a pH of 7.2 containing 0.15 M NaCl and 1% NP-40) in an amount of 10 ml/$10^8$ cells. After removal of cell residues and nucleocapside by ultracentrifugation, the solution was used as a starting material for the purification of the glycoproteins in the next step.

Lentil lectin-conjugated Sepharose® 4B (manufactured by Pharmacia of Sweden) (10 ml) was packed in a column and equilibrated with TNN buffer solution (50 mM tris · HCl, 0.5 M NaCl and 0.05% NP-40). Then 50 ml of said solution were passed through the column. After the column had been sufficiently washed with the TNN buffer solution, the glycoproteins bound to the gel were eluted with a TNN buffer solution containing 0.2 M α-methylmannoside. The eluate was fractionated and the optical density at 280 nm was measured for each fraction, whereafter the fractions with maximum absorbance were collected (20 ml). This solution showed an optical density of 1.36.

Preparation 3

Preparation of sensitized carrier:

The fixed sheep red blood cells were washed with PBS twice and the washed red blood cells were suspended in PBS at a blood cell concentration of 2.5%. The blood cells were sensitized with the antigen according to "Method in Immunology and Immunochemistry" Vol. IV, pp. 36, in the following manner: A mixture of PBS containing 0.0005% tannic acid (30 ml) was made to react with 30 ml of 25% sheep red blood cells at 37°C for 45 minutes. After the reaction, the cells were washed with PBS three times and were suspended again at a concentration of 2.5% in PBS. The purified glycoproteins prepared with lentil lectin in the above-mentioned manner were diluted with PBS so as to have an optical density at 280 nm of 0.0136. The resultant solution was mixed with an equal volume of the red blood cell suspension obtained above, and the mixture was allowed to stand at 37°C for 30 minutes for sensitization. The sensitized blood cells were washed with PBS three times and suspended in PBS containing 1% normal rabbit serum (NRS) and 0.1% sodium azide, at a concentration of the blood cells of 0.75%, to obtain 100 ml of the sensitized carrier for passive agglutination.

Example

Determination of HSV antibodies by passive agglutination with the sensitized carrier:

Each of about 120 samples of human sera was analyzed with respect to antibody titer against HSV by means of passive agglutination in the conventional manner, with the sensitized carrier produced by Preparation 3: Each sample was subjected to a sequence of two-fold dilutions with PBS containing 1% NRS on a microtiter plate. To each diluted solution were added 25 µl of the sensitized carrier and allowed to stand for 2 hours at 37°C for the observation of an agglutination pattern. The results are summarized in Table 1. Each of the numerical values in the table indicates the maximum dilution, in terms of the value "n" in the formula $2^n$, of the patterns where the agglutinations were observed. In the table there are also given the results according to the neutralizing antibody test (NT) by plaque reduction (the most common conventional method), in addition to those according to the present invention (PHA). It can be seen that all the sera samples, which are determined as being negative with respect to the antibody by NT, are also determined as negative by the PHA according to the present invention. The titers of the positive sera (in terms of the value n) determined by the PHA exhibit a close relationship with those determined by NT. Further, the PHA according to the present invention is more sensitive than NT as suggested by the high n values. Thus, the method of the present invention by PHA is useful in determining herpes simplex virus antibodies.

TABLE 1
Relationship between PHA and NT

| No. | PHA | NT | No. | PHA | NT | No. | PHA | NT |
|-----|-----|-----|-----|-----|-----|-----|-----|-----|
| 1 | 8 | 2 | 21 | 9 | 6 | 41 | 12 | 6 |
| 2 | 10 | 6 | 22 | 9 | 5 | 42 | 10 | 5 |
| 3 | 11 | 5 | 23 | 11 | 6 | 43 | <1 | <1 |
| 4 | 11 | 4 | 24 | 10 | 6 | 44 | 9 | 7 |
| 5 | <1 | <1 | 25 | <1 | <1 | 45 | 8 | 5 |
| 6 | 8 | 3 | 26 | 12 | 7 | 46 | 11 | 7 |
| 7 | 11 | 4 | 27 | 11 | 7 | 47 | <1 | <1 |
| 8 | 11 | 6 | 28 | <1 | <1 | 48 | <1 | <1 |
| 9 | 10 | 6 | 29 | 11 | 5 | 49 | <1 | <1 |
| 10 | <1 | <1 | 30 | 11 | 4 | 50 | <1 | <1 |
| 11 | 11 | 6 | 31 | <1 | <1 | 51 | 10 | 4 |
| 12 | 11 | 6 | 32 | 11 | 6 | 52 | 10 | 7 |
| 13 | 12 | 6 | 33 | 9 | 6 | 53 | 9 | 6 |
| 14 | <1 | <1 | 34 | 10 | 7 | 54 | 6 | 6 |
| 15 | 12 | 5 | 35 | 10 | 6 | 55 | <1 | <1 |
| 16 | 10 | 5 | 36 | <1 | <1 | 56 | <1 | <1 |
| 17 | <1 | <1 | 37 | <1 | <1 | 57 | 11 | 5 |
| 18 | 11 | 6 | 38 | <1 | <1 | 58 | <1 | <1 |
| 19 | <1 | <1 | 39 | 11 | 7 | 59 | 9 | 5 |
| 20 | 11 | 6 | 40 | <1 | <1 | 60 | <1 | <1 |

TABLE 1 (cont.).

| No. | PHA | NT | No. | PHA | NT | No. | PHA | NT |
|-----|-----|-----|-----|-----|-----|-----|-----|-----|
| 61 | 10 | 6 | 81 | 8 | 3 | 102 | <1 | <1 |
| 62 | 8 | 5 | 82 | 9 | 4 | 102 | <1 | <1 |
| 63 | 9 | 5 | 83 | <1 | <1 | 103 | 9 | 7 |
| 64 | <1 | <1 | 84 | 10 | 7 | 105 | <1 | <1 |
| 65 | <1 | <1 | 85 | <1 | <1 | 106 | 9 | 7 |
| 66 | <1 | 1 | 86 | <1 | <1 | 109 | <1 | <1 |
| 67 | <1 | <1 | 87 | 8 | 7 | 110 | 9 | 6 |
| 68 | <1 | <1 | 88 | 7 | 3 | 111 | 9 | 5 |
| 69 | <1 | <1 | 89 | 10 | 5 | 112 | 9 | 5 |
| 70 | 9 | 3 | 90 | 10 | 6 | 113 | 7 | 5 |
| 71 | 8 | 5 | 91 | 8 | 6 | 114 | 9 | 6 |
| 72 | 8 | 4 | 92 | 8 | 6 | 116 | 9 | 6 |
| 73 | 10 | 4 | 93 | <1 | <1 | 117 | 9 | 6 |
| 74 | 9 | 3 | 94 | 8 | 5 | 119 | <1 | <1 |
| 75 | 9 | 4 | 95 | 11 | 7 | 120 | <1 | <1 |
| 76 | 9 | 4 | 96 | <1 | <1 | 121 | 10 | 6 |
| 77 | 9 | 4 | 97 | 8 | 5 | 122 | 11 | 7 |
| 78 | 10 | 6 | 98 | 10 | 6 | 124 | 11 | 6 |
| 79 | 11 | 5 | 99 | 11 | 6 | 125 | <1 | <1 |
| 80 | 6 | 4 | 100 | <1 | <1 | 126 | <1 | <1 |
| 81 | 8 | 3 | 101 | 8 | 4 | | | |

Lyophilization of the sensitized carrier for passive hemagglutination:

The sensitized carrier suspension was subjected to the first lyophilization at 10°C under 2,66—26,6 Pa for 10 hours, and then to the second lyophilization at 25°C under 2,66—5,32 Pa for 10 hours with a lyophilizer type DF-5 (Nihon Shinku Gijutsu Corp.).

The lyophilized product was tested as to storage stability: After keeping the lyophilized sample for a fixed time (1, 3, 6, 9 and 12 months), a suitable diluent solution, for example PBS containing 1% NRS and 0.1% sodium azide, was added to the lyophilized sample to dissolve it, and then passive carrier hemagglutination reaction was carried out on positive sera.

Hardly any decrease in the titer of any of the samples could be seen and the results correlated well with the results obtained by NT. Moreover, no nonspecific reaction was found in the samples of negative sera.

## Claims

1. A method for the determination of antibodies against herpes simplex virus (HSV) which comprises sensitizing a carrier with herpes simplex virus antigens wherein said antigens are the HSV-specific glycoproteins obtained from HSV and/or cells infected with HSV, and conducting a passive agglutination assay by reacting the sensitized carrier with a sample containing the antibodies.

2. The method according to Claim 1, in which the carrier comprises fixed mammalian or avian red blood cells or an artificial carrier.

5

3. The method according to Claim 1, in which the sensitized carrier is a lyophilized product.

4. Carrier sensitized with herpes simplex virus-specific glycoproteins for carrying out the method according to any one of Claims 1 to 3.

**Patentansprüche**

1. Verfahren zur Bestimmung von Antikörpern gegen Herpes simplex-Virus (HSV), bei dem man einen Träger mit Herpes simplex-Virus-Antigenen sensitiviert, wobei die Antigene die aus HSV und/oder mit HSV infizierten Zellen erhaltenen HSV-spezifischen Glykoproteine sind, und einen passiven Agglutinations-Test durch Umsetzung des sensitivierten Trägers mit einer die Antikörper enthaltenden Probe durchführt.

2. Verfahren nach Anspruch 1, bei dem der Träger fixierte rote Blutzellen von Säugern oder Vögeln oder einen künstlichen Träger enthält.

3. Verfahren nach Anspruch 1, bei dem der sensitivierte Träger ein Gefriertrocknungsprodukt ist.

4. Träger, der mit Herpes simplex-Virus-spezifischen Glykoproteinen für die Durchführung des Verfahrens nach einem der Ansprüche 1 bis 3 sensitiviert ist.

**Revendications**

1. Un procédé pour la détermination d'anticorps contre le virus de l'herpès simplex (HSV) qui consiste à sensibiliser un support par des antigènes de virus d'herpès simplex, lesdits antigènes étant les glucoproteines spécifiques du HSV obtenues à partir de HSV et/ou de cellules infectées par le HSV, et à effectuer un test d'agglutination passive par réaction du support sensibilisé avec un échantillon contenant les anticorps.

2. Le procédé selon la revendication 1, dans lequel le support comprend des globules rouges fixés de mammifère ou d'oiseau ou un support artificiel.

3. Le procédé selon la revendication 1, dans lequel le support sensibilisé est un produit lyophilisé.

4. Support sensibilisé par des glucoprotéines spécifiques du virus de l'herpés simplex pour la mise en oeuvre du procédé selon l'une quelconque des revendications 1 à 3.